# EUROPEAN PATENT SPECIFICATION

(11) **EP 1 250 125 B1**
(45) Date of publication and mention of the grant of the patent: **03.12.2003**
(21) Application number: 01925320.2
(22) Date of filing: 25.01.2001
(51) Int. Cl.: A61K 9/107, A61K 31/135

(54) **PHARMACEUTICAL COMPOSITIONS COMPRISING TERBINAFINE**
PHARMAZEUTISCHE ZUSAMMENSETZUNGEN, DIE TERBINAFIN ENTHALTEN
COMPOSITIONS PHARMACEUTIQUES CONTENANT DE LA TERBINAFINE

(30) Priority: 27.01.2000 GB 0001928
(43) Date of publication of application: 23.10.2002
(73) Proprietor: Novartis AG, 4056 Basel (CH); Novartis Pharma GmbH, 1230 Wien (AT)
(72) Inventor: BONNY, Jean-Daniel, CH-4414 Füllinsdorf (CH); EUGSTER, Andreas, Carl, CH-4123 Allschwil (CH); GUITARD, Patrice, F-68220 Hégenheim (FR); HIRSCH, Stefan, 79539 Lörrach (DE)
(74) Representative: Gros, Florent
(86) International application number: EP0100834
(87) International publication number: WO01054675

(56) References cited:
- WO-A-00/59475
- WO-A-01/01960

## Description

The present invention relates to a pharmaceutical composition comprising terbinafine.

Terbinafine may be used e.g. in free form or in acid addition salt form. An acid addition salt form may be prepared from the free base form in conventional manner and vice-versa. Examples of suitable acid addition salt form are the hydrochloride, the lactate and the ascorbate. The free base and the hydrochloride are preferred.

Terbinafine is known from e.g. BE-PS-853976 and EP-A-24587. It belongs to the class of allylamine anti-mycotics. It is acknowledged in the art and is commercially available under the trade name Lamisil®.

Terbinafine is highly active upon both topical and oral administration. While numerous pharmaceutical compositions for topical and oral administration have been proposed, there still exists a need for commercially acceptable liquid terbinafine formulations for oral administration, especially for oral administration to children. One particular difficulty in the formulation of terbinafine in liquid pharmaceutical compositions is its unpleasant, e.g. bitter, taste and/or low physical stability.

It is thus desirable to provide liquid oral terbinafine formulations with a high physical stability and acceptable taste.

A promising approach overcoming the above-mentioned difficulties has now been found in the form of emulsions comprising terbinafine as the active agent. Thus pharmaceutically acceptable liquid oral dosage forms of high physical stability and acceptable taste have been obtained by formulating pharmaceutical compositions comprising terbinafine in the form of emulsions.

Accordingly in one aspect the present invention provides a terbinafine pharmaceutical composition which is emulsifiable or self-emulsifying or in form of an emulsion, e.g. an oil in water or water in oil emulsion, preferably an oil in water emulsion, wherein the composition is adapted for oral administration, and comprising a lipophilic component, a surfactant and an emulsion stabilizing agent, e.g. an agent preventing breakdown, e.g. creaming, coalescence or sedimentation, of the emulsion.

The compositions of the present invention comprise terbinafine preferably in base form.

Terbinafine may be present in an amount of 0.1 to 10 %, e.g. 1 to 5%, preferably 1.5 to 4% by weight based on the total weight of the composition.

Suitable lipophilic components according to the present invention are:
(i) mono-, and/or di-, and/or tri, e.g. C₆₋₂₄, e.g. C₆₋₂₀, e.g. C₈₋₁₈, e.g. saturated and/or mono- or di-unsaturated fatty acid esters of glycerol, e.g.
   a) monoglycerides, of e.g. C₈-C₁₀ fatty acids, e.g. as known and commercially available under the trade name Imwitor® 308, or Imwitor® 310 e.g. from Condea, and/or
   b) diglycerides, of e.g. C₈-C₁₀ fatty acids, e.g. C₈, e.g. as known and commercially available under the trade name Sunfat® GDC-N e.g. from Taiyo Kagaku Co., and/or
   c) a mixture of mono-, diglycerides, of e.g. C₈-C₁₀ fatty acids, e.g. as known and commercially available under the trade name Imwitor® 742, or Capmul® MCM e.g. from Condea or Abitec Corp., or with e.g. a monoglyceride of C₁₈ fatty acid as its main component, e.g. as known and commercially available under the trade name GMOrphic®-80 or Tegin® O e.g. from Eastman Co. or Goldschmidt Co., and/or
   d) mixed mono-, di-, tri-glycerides, of e.g. C₁₆-C₁₈ fatty acids, e.g. as known and commercially available under the trade name Maisine® e.g. from Gattefossé, and/or
   e) medium chain fatty acid triglycerides, of e.g. C₆-C₁₂ fatty acids, e.g. as known and commercially available under the trade name Acomed®, Myritol®, Captex®, Neobee®M5F, Miglyol®810, Miglyol®812, Miglyol®818, Mazol®, Sefsol®860, Sefsol®870 e.g. from Condea or Stepan Europe, and/or
ii) mono-, and/or di-, and/or tri, e.g. C₆₋₂₄, e.g. C₆₋₂₀, e.g. C₈₋₁₈, e.g. saturated and/or mono- or di-unsaturated fatty acid esters of propylene glycol, e.g.
   a) propylene glycol mono fatty acid esters, e.g. C₆-C₁₈ fatty acids, e.g. propylene glycol hydroxystearate, propylene glycol isostearate, propylene glycol ricinoleate, propylene glycol stearate, propylene glycol laurate, e.g. as known and commercially available under the trade name Lauroglycol® 90, Sefsol® 218, or Capryol® 90 e.g. from Nikko Chemicals Co. or Gattefossé, and/or
   b) propylene glycol di fatty acid esters, e.g. C₈-C₁₂ fatty acids, e.g. propylene glycol dicaprylate, e.g. as known and commercially available under the trade name Miglyol® 840 e.g. from Condea, or propylene glycol dilaurate, and/or
iii) transesterified ethoxylated vegetable oils, e.g. as known and commercially available under the trade name Labrafil® e.g. from Gattefossé, and/or
iv) fatty acids or alcohols, e.g. C₆₋₂₄, e.g. C₆₋₂₀, e.g. C₈₋₁₈, e.g. saturated and/or mono- or di-unsaturated, e.g. oleic acid, oleyl alcohol, linoleic acid, capric acid, caprylic acid, caproic acid, tetradecanol, dodecanol, decanol, and/or
v) esterified compounds of fatty acid and primary alcohol, e.g. C₆₋₂₄, e.g. C₆₋₂₀, e.g. C₈₋₁₈, fatty acids and C₂-C₃ alcohols, e.g. isopropyl myristate, isopropyl palmitate, ethyl linoleate, ethyl oleate
vi) pharmaceutically acceptable oils, e.g. soybean oil, peanut oil, or olive oil.

Particularly suitable are triglycerides of e.g. C₆₋₁₂, e.g. saturated fatty acids, e.g. as known and commercially available under the trade name Miglyol®812. Miglyol®812 is a fractionated coconut oil comprising caprylic-capric acid triglycerides and having a molecular weight of about 520 daltons. Fatty acid composition = C₆ max. about 3%, C₈ about 50 to 65%, C₁₀ about 30 to 45%, C₁₂ max 5%; acid value about 0.1; saponification value about 330 to 345; iodine value max 1. Miglyol® 812 is available from Condea.

The lipophilic component may be present in an amount of 5 to 50 %, e.g. 10 to 40%, preferably 15 to 25% by weight based on the total weight of the composition.

In a further aspect the present invention provides a pharmaceutical composition according to the present invention wherein the ratio of terbinafine : lipophilic component may be 1 : 1 to 20, e.g. 1 : 2 to 15, preferably 1 : 4 to 10.

Examples of suitable surfactants for use in this invention are:
i) A positively or negatively charged or a polar surfactant having C₁₂₋₂₂ saturated or mono- or di-unsaturated alkyl groups, especially an electrically charged phosphatide, being no zwitterion, preferably a negatively charged phosphatide. Preferably a phospholipid, e.g. a lecithin may be used. Suitable lecithins include e.g. soy bean- or egg-lecithin as known and commercially available under the trade names Phospholipon® 80 or Lipoid® E 80, e.g. from Lipoid. It contains apart from ist preponderant component phosphatidylcholine with a zwitterion structure also other, negatively charged and polar components. (Fiedler, loc. cit., 2, p. 910, 1184).
ii) Polyoxyethylene-sorbitan- C₈₋₂₀ fatty acid esters (polysorbates) e.g. produced by copolymerising ethylene oxide with fatty acid esters of a sorbitol and its anhydrides of e.g. mono- and tri- lauryl, palmityl, stearyl and oleyl esters e.g. of the type known and commercially available under the trade name Tween@ (Fiedler, loc.cit., 2, pp.1615) including the products Tween@
   20 [polyoxyethylene(20)sorbitanmonolaurate],
   21 [polyoxyethylene(4)sorbitanmonolaurate],
   40 [polyoxyethylene(20)sorbitanmonopalmitate],
   60 [polyoxyethylene(20)sorbitanmonostearate],
   65 [polyoxyethylene(20)sorbitantristearate],
   80 [polyoxyethylene(20)sorbitanmonooleate],
   81 [polyoxyethylene(5)sorbitanmonooleate],
   85 [polyoxyethylene(20)sorbitantrioleate].
   Especially preferred products of this class are Tween® 40 and Tween® 80.
iii) Sorbitan C₈₋₂₀ fatty acid esters, e.g. sorbitan mono C₁₂₋₁₈ fatty acid esters, or sorbitan tri C₁₂₋₁₈ fatty acid esters as known and commercially available under the trade mark Span® from e.g. ICI. An especially preferred product of this class is e.g. Span@ 20 (sorbitan monolaurate) or Span® 80 (sorbitan monooleate) (Fiedler, loc. cit., 2, p. 1430; Handbook of Pharmaceutical Excipients, loc. cit., page 473).
iv) Polyoxyethylene C₈₋₂₀ fatty acid esters, for example polyoxyethylene stearic acid esters of the type known and commercially available under the trade name Myrj® (Fiedler, loc. cit., 2, p. 1042). An especially preferred product of this class is Myrj® 52 having a D²⁵ of about 1.1, a melting point of about 40 to 44°C, an HLB value of about 16.9, an acid value of about 0 to 1 and a saponification value of about 25 to 35.
v) Polyoxyethylene C₈₋₂₀ alkyl ethers, e.g. polyoxyethylene glycol ethers of C₁₂ to C₁₈ alcohols, e.g. Polyoxyl 2-, 10- or 20-cetyl ether or Polyoxyl 4- or 23-lauryl ether, or polyoxyl 2-, 10- or 20-oleyl ether, or Polyoxyl 2-, 10-, 20- or 100-stearyl ether, as known and commercially available under the trade mark Brij® from e.g. ICI. An especially preferred product of this class is e.g. Brij® 35 (Polyoxyl 23 lauryl ether) or Brij@ 98 (Polyoxyl 20 oleyl ether) (Fiedler, loc. cit., 1, pp. 259; Handbook of Pharmaceutical Excipients, loc. cit., page 367).
   Similar products which may also be used are polyoxyethylene-polyoxypropylene- C₈₋₂₀ alkyl ethers, e.g. polyoxyethylene-polyoxypropylene- ethers of C₁₂ to C₁₈ alcohols, e.g. polyoxyethylen-20-polyoxypropylene-4-cetylether which is known and commercially available under the trade mark Nikkol PBC® 34, from e.g. Nikko Chemicals Co., Ltd. (Fiedler, loc. cit., vol. 2, pp. 1239).
vi) reaction products of a natural or hydrogenated castor oil and ethylene oxide. The natural or hydrogenated castor oil may be reacted with ethylene oxide in a molar ratio of from about 1:35 to about 1:60, with optional removal of the polyethyleneglycol component from the products. Various such surfactants are commercially available. The polyethyleneglycol-hydrogenated castor oils available under the trade name Cremophor® are especially suitable. Particularly suitable is Cremophor® RH40, which has a saponification value of about 50 to 60, an acid value less than about 1, a water content (Fischer) less than about 2%, an n_{D}⁶⁰ of about 1.453 to 1.457 and an HLB of about 14 to 16; and Cremophor® RH 60, which has a saponification value of about 40 to 50, an acid value less than about 1, an iodine value of less than about 1, a water content (Fischer) of about 4.5 to 5.5%, an n_{D}⁶⁰ of about 1.453 to 1.457 and an HLB of about 15 to 17. Also suitable are polyethyleneglycol castor oils such as those available under the trade names Cremophor® EL, which has a molecular weight (by steam osmometry) of about 1630, a saponification value of about 65 to 70, an acid value of about 2, an iodine value of about 28 to 32 and an n_{D}²⁵ of about 1.471; or Simulsol® OL-50 (PEG-40 castor oil), having a saponification value of about 55 to 65, an acid value of max. 2, an iodine value of 25 to 35, a water content of max. 8%, and an HLB of about 13, available from Seppic.
   Similar or identical products which may also be used are available under the trade names Nikkol® (e.g. Nikkol® HCO-40 and HCO-60), Mapeg® (e.g. Mapeg® CO-40h), Incrocas® (e.g. Incrocas® 40), Tagat® (for example polyoxyethylene-gtycerol-fatty acid esters, e.g. Tagat@ RH 40, and Tagat@ TO, a polyoxyethylene-glycerol-trioleate having a HLB value of 11.3). These surfactants are further described in Fiedler loc. cit.
vii) Polyoxyethylene mono esters of a saturated C₁₀₋₂₂, e.g. C₁₈ substituted e.g. hydroxy fatty acid; e.g. 12 hydroxy stearic acid PEG ester, e.g. of PEG about e.g. 600-900 e.g. 660 daltons MW, e.g. Solutol® HS 15 from BASF, Ludwigshafen, Germany.
viii) Polyoxyethylene-polyoxypropylene co-polymers and block co-polymers, poloxamers, for example of the type known and commercially available under the trade names Pluronic®, Emkalyx® (Fiedler, loc. cit., 2, p. 1203). An especially preferred product of this class is Pluronic® F68 (poloxamer 188), having a melting point of about 52°C and a molecular weight of about 6800 to 8975. A further preferred product of this class is Synperonic® PE L44 (poloxamer 124).
ix) Dioctylsodiumsulfosuccinate as known and commercially available under the trade mark Aerosol OT® from e.g. American Cyanamid Co. (Fiedler, loc. cit., 1, p. 118), or di-[2-ethylhexyl]-succinate (Fiedler, loc. cit., 1, p. 487).
x) Water soluble tocopheryl polyethylene glycol succinic acid esters (TPGS), e.g. with a polymerisation number from about 500 to about 2000, e.g. of about 1000, e.g. available from Eastman Fine Chemicals Kingsport, Texas, USA.
xi) Polyglycerol fatty acid esters, with e.g. from 2 to 20, e.g. 10 glycerol units. The fatty acid constituent may include both saturated and unsaturated fatty acids having a chain length of from e.g. C₈₋₂₀. Particularly suitable is e.g. decaglycerylmonolaurat or decaglycerylmonomyristat, as known and commercially available under the trade mark Decaglyn® 1-L or Decaglyn® 1-M, respectively, from e.g. Nikko Chemicals C., Ltd (Fiedler, loc. cit., vol. 2, pp. 1228).
xii) Polyethylene glycol glyceryl fatty acid ester. The fatty acid ester may include mono and/or di and/or tri fatty acid ester. The fatty acid constituent may include both saturated and unsaturated fatty acids having a chain length of from e.g. C₈-C₂₀. The polyethylene glycols may have e.g. from 5 to 40 [CH₂-CH₂-O] units, e.g. 5 or 30 units. Particularly suitable is polyethylene glycol (15) glyceryl monostearat or polyethylene glycol (15) glyceryl monooleate which is commercially available, e.g. under the trade name TGMS®-15 or TGMO®-15, respectively, e.g. from Nikko Chemicals Co., Ltd. Further suitable is polyethylene glycol (30) glyceryl monooleate which is commercially available, e.g. under the trade name Tagat® O, e.g. from Goldschmidt (H. Fiedler, loc cit, vol. 2, p. 1502-1503). Further suitable are polyethylene glycol glyceryl C₈-C₁₀ fatty acid ester with from 5 to 10 [CH₂-CH₂-O] units, e.g. 7 units, e.g. Cetiol® HE, or Labrasol®.
xiii) Sterols and derivatives thereof, for example cholesterols and derivatives thereof, in particular phytosterols, e.g. products comprising sitosterol, campesterol or stigmasterol, and ethylene oxide adducts thereof, for example soy bean sterols and derivatives thereof, e.g. polyethylene glycol sterols, e.g. polyethylene glycol phytosterols or polyethylene glycol soy bean sterols. The polyethylene glycols may have e.g. from 10 to 40 [CH₂-CH₂-O] units, e.g. 25 or 30 units. Particularly suitable is polyethylene glycol (30) phytosterol which is commercially available, e.g. under the trade name Nikkol BPS®-30, e.g. from Nikko Chemicals Co., Ltd. Further suitable is polyethylene glycol (25) soy bean sterol which is commercially available, e.g. under the trade name Generol® 122 E 25, e.g. from Henkel (H. Fiedler, loc cit, vol. 1, p. 680).
xiv) Sodium lauryl sulfate, which has a molecular weight of 288.38, is freely soluble in water and has an HLB value of about 40. It may be obtainable by sulfation of lauryl alcohol, followed by neutralization with sodium carbonate (Handbook of Pharmaceutical Excipients, loc. cit., pp 448).

It is to be appreciated that surfactants may be complex mixtures containing side products or unreacted starting products involved in the preparation thereof, e.g. surfactants made by polyoxyethylation may contain another side product, e.g. polyethylene glycol.

A surfactant having a hydrophilic-lipophilic balance (HLB) value of 8 to 17, e.g. 10 to 12 is preferred. The HLB value is preferably the mean HLB value.

Particularly suitable for the compositions of this invention are phospholipids, e.g. soy bean- or egg-lecithin, or polyoxyethylene sorbitan fatty acid ester and/or sorbitan fatty acid esters.

It is to be appreciated that according to the present invention one or a mixture of the above-mentioned surfactants can be used. When the surfactant is a mixture of polyoxyethylene(20)sorbitanmonooleate (Tween® 80) and sorbitanmonolaurate (Span@ 20), the weight ratio of Tween 80 : Span 20 is e.g. 1 to 10 : 1 to 10, e.g.1 to 4 : 1 to 4, e.g. 1 part by weight Tween 80 is used per 4 parts by weight of Span 20, or 4 parts by weight Tween 80 are used per 1 part by weight of Span 20.

The surfactant may be present in an amount of 0.2 to 20%, e.g. 1 to 10%, preferably 1.5 to 5% by weight based on the total weight of the composition.

In a further aspect the present invention provides a pharmaceutical composition according to the present invention wherein the ratio of terbinafine : surfactant may be 1 : 0.05 to 10, e.g. 1 : 0.1 to 5, preferably 1 : 0.5 to 2.

As emulsion stabilizing agents, e.g. agents preventing breakdown, e.g. creaming, coalescence or sedimentation, of the emulsion, e.g. thickening, e.g. viscosity increasing, agents, may be used. Suitable thickening, e.g. viscosity increasing, agents may be of those known and employed in the art, including e.g. pharmaceutically acceptable polymeric materials and inorganic thickening agents, e.g. thixotropic agents, for example, of the following types:
i) Water soluble celluloses and cellulose derivatives including: alkyl celluloses, e.g. methyl-, ethyl- and propyl-celluloses; hydroxyalkyl-celluloses, e.g. hydroxypropyl-celluloses and hydroxypropylalkyl-celluloses such as hydroxypropyl-methyl-celluloses; and salts thereof such as sodium-carboxymethyl-celluloses. Examples of such products suitable for use in accordance with the present invention are those known and commercially available, e.g. under the trade names Klucel® and Methocel® (c.f. Fiedler, loc. cit., pp. 855 and 986). Particularly suitable may be a mixture of sodium carboxymethylcellulose and microcrystalline cellulose as known and commercially available under the trade name e.g. Avicel® RC 591 from e.g. FMC Corporation Food & Pharmaceutical Products.
ii) Water soluble poiyvinylpyrrolidones, including for example poly-N-vinylpyrrolidones and vinylpyrrolidone co-polymers such as vinylpyrrolidone-vinylacetate co-polymers, especially of low molecular weight. Examples of such compounds suitable for use in accordance with the present invention are those known and commercially available, e.g. under the trade name Kollidon® (or, in the USA, Povidone®) (c.f. Fiedler, loc. cit., 1, pp. 864), in particular the products Kollidon® 30 and 90;
iii) Polyacrylic acid, as known under the names carboxypolymethylen, or carboxyvinylpolymer, or carbomer, or Carbopol®, e.g. with a molecular weight of from about 860 000 to about 1 000 000, obtainable by polymerisation of acrylic acid (H.P. Fiedler, loc. cit., 2, p. 1204).
iv) Inorganic thickening agents such as atapulgite, bentonite and silicates including hydrophilic silicon dioxide products, e.g. alkylated, for example methylated, silica gels, in particular colloidal silicon dioxide products as known and commercially available under the trade name Aerosil® (c.f. H.P. Fiedler, loc. cit., 1, pp. 115; Handbook of Pharmaceutical Excipients, loc. cit., pp. 424) in particular the products Aerosil® 130, 200, 300, 380, O, OX 50, TT 600, MOX 80, MOX 170, LK 84 and the methylated Aerosil® R 972.

Further suitable for stabilization of the pharmaceutical compositions of this invention is high pressure homogenization. Addition of a thickening agent may be superfluous in these compositions.

Accordingly, in one aspect the present invention provides an emulsifiable terbinafine pharmaceutical composition adapted for oral administration which upon high pressure homogenization forms an emulsion.

The thickening agent, when present, may be present in an amount of 0.1 to 10%, e.g. 0.5 to 5%, preferably 0.75 to 3% by weight based on the total weight of the composition.

In a further aspect the present invention provides a pharmaceutical composition according to the present invention wherein the ratio of terbinafine : emulsion stabilizing agent may be 1 : 0.05 to 10, e.g. 1 : 0.1 to 5, preferably 1 : 0.2 to 2.

Any carbon chain not otherwise specified herein conveniently contains 1 to 18 carbon atoms, e.g. 10 to 18 carbon atoms, when a terminal group or 2 or 3 carbon atoms when a polymer moiety.

It will be appreciated that the present invention encompasses
a) in respect of the lipophilic component any of components i) to vi) individually or in combination with one, two or more of the other components i) to vi),
b) in respect of the surfactant any of the surfactants specified above, e.g. surfactants i) to xiv), individually or in combination
c) in respect of the emulsion stabilizing agent any of the components specified above, e.g. components i) to iv), individually or in combination.

The compositions of the present invention may comprise water, e.g. purified water, in amount of 20 to 80%, e.g. 30 to 60%, preferably 35 to 55% by weight based on the total weight of the composition.

In another aspect of the present invention the terbinafine pharmaceutical composition may be a self-emulsifying composition which on administration to water may form an emulsion.

The compositions may also include one or more further additives or ingredients e.g. in an amount of from 0.01 to 15% by weight based on the total weight of the composition, in particular anti-oxidants, e.g. ascorbyl palmitate, butylated hydroxy anisole (BHA), butylated hydroxy toluene (BHT), or tocopherols, preferabyl DL-alpha-tocopherol (vitamin E); preserving agents, e.g. sorbic acid, potassium sorbat, benzoic acid, or parabenes, e.g. methyl-, propylparabene, preferably sorbic acid; sweetening agents, e.g. sorbitol, saccharin, acesulfam, aspartame, or sugars, e.g. glucose, fructose or saccharose, preferably sorbitol is used, e.g. in form of an aqueous solution which contains 70% sorbitol by weight; flavouring agents, e.g. banana, strawberry, vanilla or chocolate flavour, and so forth.

Determination of workable proportions in any particular instance will generally be within the capability of the man skilled on the art. All indicated proportions and relative weight ranges described above are accordingly to be understood as being indicative of preferred or individually inventive teachings only and not as limiting the invention in its broadest aspect.

Preferably the pH value of the composition of this invention may be e.g. about 4 to 8, e.g. about 5 to 7, preferably about 6.

Details of excipients of the invention are described in Fiedler, H. P. "Lexikon der Hilfsstoffe für Pharmazie, Kosmetik und angrenzende Gebiete", Editio Cantor Verlag Aulendorf, Aulendorf, 4th revised and expanded edition (1996); "Handbook of Pharmaceutical Excipients", 2nd Edition, Editors A. Wade and P. J. Weller (1994), Joint publication of American Pharmaceutical Association, Washington, USA and The Pharmaceutical Press, London, England; or may be obtained from the relevant manufacturers, the contents of which are hereby incorporated by reference.

In a further alternative aspect the invention also provides a process for the production of a pharmaceutical composition as defined above, which process may comprise bringing the lipophilic phase comprising the lipophilic component, the lipophilic surfactant, when present, and terbinafine and the aqueous phase comprising water and the hydrophilic surfactant, when present, into intimate admixture to prepare an emulsion.

Optionally further components or additives, in particular antioxidants, and/or preserving agents, and/or sweetening agents, and/or flavouring agents, and/or thickening agents may be mixed with either the lipophilic phase or the aqueous phase or may be added to the final product as appropriate.

In a further aspect the present invention provides a composition according to the present invention wherein the mean droplet size of lipophilic phase, e.g. fat, e.g. containing the active agent, may be about 0.1 to about 30 µm, e.g. about 0.5 to about 15 µm, e.g. about 0.8 to about 10 µm.

The composition according to the present invention conveniently is an emulsion for oral administration. Naturally as with any emulsion the excipients may increase the viscosity of the composition or the oil and water phases may separate, e.g. creaming or coalescence may be observed. Just before use the compositions may be shaken.

The compositions of the invention may be used directly or after dilution with e.g. milk or juice and administered as a liquid. The compositions may also be administered in a capsule.

In a further aspect the present invention provides a method of orally administering a pharmaceutical composition, said method comprising orally administering to a patient in need of terbinafine therapy a composition according to the present invention.

The present invention provides a composition of surprisingly high physical stability, e.g. for up to two or more years, and surprisingly acceptable taste. The physical stability may be tested as conventional, e.g. the compositions may be tested as such by microscopy, or the droplet size may be determined after dilution of the composition by laser light diffraction, e.g. after storage at room temperature, i.e. at 25°C, and/or after storage at 40°C. The taste of the compositions may be tested in standard clinical studies.

The compositions of this invention are useful for the known indications of terbinafine, e.g. for the following conditions: onychomycosis caused by dermatophyte fungi, tinea capitis, fungal infections of the skin, for the treatment of tinea corporis, tinea cruris, tinea pedis, and yeast infections of the skin caused by the genus Candida, e.g. Candida albicans, systemic mycosis, mycosis by azole-resistant strains, e.g in combination with a 14-alpha-methyldimethylase inhibitor, or infections with Helicobacter pylori.

The composition is particularly effective in treating tinea capitis in e.g. children.

In a further aspect the present invention provides a method for the treatment of fungal infections of the human body comprising administering a pharmaceutically effective amount of a pharmaceutical composition according to the present invention to a subject in need of such treatment.

In yet a further aspect the present invention provides the use of a composition according to the present invention in the manufacture of a medicament for the treatment or fungal infections of the human body.

The utility of the pharmaceutical compositions of the present invention may be observed in standard bioavailability tests or standard animal models, for example ascertaining dosages of the present compositions giving blood levels of terbinafine equivalent to blood levels giving a therapeutical effect on administration of known terbinafine oral dosage forms, e.g. a tablet. Typical doses are in the range of 1 mg/kg to 10 mg/kg, e.g. 1.5 mg/kg to 5 mg/kg, or e.g. 3 to 4 mg/kg body weight of terbinafine per day.

The appropriate dosage will, of course, vary depending upon, for example, the host and the nature and severity of the condition being treated. However in general satisfactory results in animals are indicated to be obtained at daily treatments with doses from about 1 mg/kg to about 10 mg/kg animal body weight. In humans an indicated daily dosage is in the range from about 10 mg to about 1000 mg per day, conveniently administered, for example, in divided doses up to four times a day or once daily. Preferred dosages for children weighing < 20 kg may be about 62.5 mg once daily, for children weighing 20 to 40 kg about 125 mg once daily, for children weighing > 40 kg about 250 mg once daily, and for adults from about 250 mg to about 500 mg once daily.

Following is a description by way of example only of compositions of the invention.

### Examples 1 to 3

Compositions according to the invention and of the following examples were prepared by mixing the ingredients and homogenizing with the help of a high shear rotor-stator.

| **Component** | **Example 1** (g/100 ml) | **Example 2** (g/100 ml) | **Example 3** (g/100 ml) |
|---|---|---|---|
| Terbinafine base | 2.50 | 2.50 | 2.50 |
| Miglyol® 812 | 20.00 | 20.00 | 20.00 |
| Lipoid® E80 | 2.00 | - | - |
| Tween® 80 | - | 0.50 | 2.00 |
| Span® 20 | - | 2.00 | 0.50 |
| Avicel® RC 591 | 1.20 | 1.20 | 1.20 |
| DL-alpha tocopherol | 0.02 | 0.02 | 0.02 |
| Sorbic acid | 0.10 | 0.10 | 0.10 |
| Potassium sorbate | 0.10 | 0.10 | 0.10 |
| Sorbitol solution 70%(w/w) | 20.00 | 20.00 | 20.00 |
| Flavouring agent | 0.25 | 0.25 | 0.25 |
| Purified water | ad 100 ml | ad 100 ml | ad 100 ml |

The following publications have been cited:
- WO 0101960;
- WO 0059475.

They have been published after the priority date of the present invention and do not anticipate the novelty of the present subject matter.

## Claims

1. A terbinafine pharmaceutical composition which is emulsifiable or self-emulsifying or in form of an emulsion wherein the composition is adapted for oral administration, comprising a lipophilic component, a surfactant and an emulsion stabilizing agent.

2. A pharmaceutical composition according to claim 1 wherein the lipophilic component is a triglyceride of C₆₋₁₂ saturated fatty acids.

3. A pharmaceutical composition according to claim 1 wherein the lipophilic component is present in an amount of 15 to 25% by weight based on total weight of composition.

4. A pharmaceutical composition according to claim 1 wherein the surfactant is a phospholipid.

5. A pharmaceutical composition according to claim 1 wherein the surfactant is present in an amount of 1.5 to 5% by weight based on total weight of composition.

6. A pharmaceutical composition according to claim 1 wherein the emulsion stabilizing agent is a mixture of sodium carboxymethylcellulose and microcrystalline cellulose.

7. A pharmaceutical composition according to claim 1 further comprising water.

8. A pharmaceutical composition according to claim 1 wherein the mean droplet size of lipophilic phase is of about 0.1 to 30 µm.

## Patentansprüche

1. Terbinafin enthaltende pharmazeutische Zusammensetzung, die emulgierbar oder selbst emulgierbar ist oder in Form einer Emulsion vorliegt, worin die Zusammensetzung für eine orale Verabreichung angepasst ist und eine lipophile Komponente, ein Tensid und einen Emulsionsstabilisator umfasst.

2. Pharmazeutische Zusammensetzung nach Anspruch 1, worin die lipophile Komponente ein Triglycerid einer gesättigten C₆-C₁₂ Fettsäure ist.

3. Pharmazeutische Zusammensetzung nach Anspruch 1, worin die lipophile Komponente in einer Menge von 15 bis 25 Gew.-%, bezogen auf das Gesamtgewicht der Zusammensetzung, vorhanden ist.

4. Pharmazeutische Zusammensetzung nach Anspruch 1, worin das Tensid ein Phospholipid ist.

5. Pharmazeutische Zusammensetzung nach Anspruch 1, worin das Tensid in einer Menge von 1,5 bis 5 Gew.-%, bezogen auf das Gesamtgewicht der Zusammensetzung, vorhanden ist.

6. Pharmazeutische Zusammensetzung nach Anspruch I, worin der Emulsionsstabilisator ein Gemisch aus Natriumcarboxymethylcellulose und mikrokristalliner Cellulose ist.

7. Pharmazeutische Zusammensetzung nach Anspruch 1, die femer auch Wasser enthält.

8. Pharmazeutische Zusammensetzung nach Anspruch 1, worin die mittlere Tröpfchengröße der lipophilen Phase etwa 0,1 bis 30 µm beträgt.

## Revendications

1. Composition pharmaceutique de terbinafine qui est émulsifiable ou auto-émulsifiante ou sous la forme d'une émulsion dans laquelle la composition est adaptée pour l'administration par voie orale, comprenant un composant lipophile, un tensio-actif et un agent stabilisant d'émulsion.

2. Composition pharmaceutique selon la revendication 1 dans laquelle le composant lipophile est un triglycéride d'acides gras saturés C₆₋₁₂.

3. Composition pharmaceutique selon la revendication 1 dans laquelle le composant lipophile est présent dans une quantité de 15 à 25% en poids sur la base du poids total de la composition.

4. Composition pharmaceutique selon la revendication 1 dans laquelle le tensio-actif est un phospholipide.

5. Composition pharmaceutique selon la revendication 1 dans laquelle le tensio-actif est présent dans une quantité de 1,5 à 5% en poids sur la base du poids total de la composition.

6. Composition pharmaceutique selon la revendication 1 dans laquelle l'agent stabilisant d'émulsion est un mélange de carboxyméthylcellulose sodique et de cellulose microcristalline.

7. Composition pharmaceutique selon la revendication 1. comprenant de plus de l'eau.

8. Composition pharmaceutique selon la revendication 1 dans laquelle la dimension de la gouttelette moyenne de la phase lipophilique est d'environ 0,1 à 30 µm.
